(19) Europäisches Patentamt
European Patent Office
Office européen des brevets

(11) **EP 3 182 119 A1**

(12) **EUROPEAN PATENT APPLICATION**

(43) Date of publication:
**21.06.2017 Bulletin 2017/25**

(51) Int Cl.:
***G01N 33/28*** *(2006.01)*

(21) Application number: **15290314.2**

(22) Date of filing: **15.12.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Geoservices Equipements SAS
95971 Roissy en France (FR)**

(72) Inventors:
• **Colombel, Emilie
95971 Roissy (FR)**

• **Guerriero, Nicolas
95971 Roissy (FR)**
• **Breviere, Jerome
95971 Roissy (FR)**

(74) Representative: **Leonori, Céline
Etudes et Productions Schlumberger
Intellectual Property Department
1, rue Henri Becquerel
B.P. 202
92142 Clamart Cedex (FR)**

(54) **METHOD OF DETERMINING THE CONTENT OF AT LEAST ONE COMPOUND CONTAINED IN A DRILLING MUD**

(57)    Method of determining the content of at least one drilling compound in a drilling mud, comprising:
- obtaining a first flow of said drilling mud exiting a wellbore,
- performing a first gas extraction by injecting at least a first part of the first flow in a first extraction device (53A) and extracting gas from the first extraction device in order to obtain a first extracted gas and a second flow of drilling mud, and measuring at least a first parameter representative of a gas fraction of said drilling compound in the first extracted gas, and

- using at least the first parameter and a correction factor in order to calculate said content;
- performing a second gas extraction in a second extraction device (53B) distinct from the first extraction device in order to obtain at least a second extracted gas and a third flow of drilling mud, and measuring at least a second parameter representative of a composition of the second extracted gas, and
- using at least the second parameter in order to calculate the correction factor.

FIG.2

EP 3 182 119 A1

## Description

The present disclosure deals with a method of determining the content of at least one compound contained in a drilling mud, the method comprising:

- obtaining a first flow of said drilling mud exiting a well bore,
- performing a first gas extraction by injecting at least a first part of the first flow in a first extraction device and extracting gas from the first extraction device in order to obtain a first extracted gas and a second flow of drilling mud exiting the first extraction device, and measuring at least a first parameter representative of a gas fraction of said drilling compound in the first extracted gas, and
- using at least the first parameter and a correction factor in order to calculate said content of the drilling compound.

The present disclosure also relates an analysis assembly suitable for performing the method.

During the drilling of a petroleum or gas well, it is known how to perform an analysis of the gas drilling compounds contained in the drilling fluid emerging from the well, this fluid being commonly designated as "drilling mud".

This analysis gives the possibility of reconstructing the geological succession of the crossed layers of the formation during the drilling and is involved in the determination of the opportunities of exploiting fluids contained in the formation.

This analysis performed continuously comprises two main phases. A first phase consists in continuously sampling the drilling mud in circulation, and then in bringing it into an extraction enclosure where a certain number of drilling compounds carried by the mud (for example hydrocarbon compounds, carbon dioxide, hydrogen sulfide, helium and nitrogen) are extracted from the mud as a gas.

A second phase consists in transporting the extracted gases towards an analyzer where these composition of these gases are determined and in certain cases, where each of these gases is quantified.

For extracting the gases from the mud, a degasser with mechanical stirring of the type described in FR 2 799 790 is frequently used.

The gases extracted from the mud, mixed with a carrier gas introduced into the degasser are conveyed by suction through a gas extraction conduit up to an analyzer which allows quantification of the extracted gases.

With such a device it is possible to significantly and specifically extract the very volatile gases present in the mud, for example the $C_1$-$C_5$ hydrocarbons, notably when it is used with a device for heating the drilling mud, placed upstream of the degasser or in the latter.

However the extraction, in the degasser, of the drilling compounds contained in the mud is not total and the extraction efficiency, defined as the amount of an extracted drilling compound referred to the total amount of this same drilling compound initially contained in the mud, depends on the nature of the drilling compound. A method for empirically correcting the measurement carried on the gas fraction extracted for each drilling compound with a correction factor depending on the drilling compound in order to provide an estimate of the actual content of the drilling compound in the drilling mud is known in the art.

EP-A-1 710 575 describes such a method, wherein a same calibration sample of the drilling fluid, containing calibration compounds, successively undergoes several extraction stages in the degasser, the amount of extracted gas being measured at each extraction stage. This method is known as "multi-pass" calibration method.

On the basis of the gas fractions measured at each extraction stage for each calibration compound, a correction factor relating the content of a given calibration compound to the measured fraction during a first extraction stage in the degasser may be determined experimentally for each calibration compound. In order to apply it, it is necessary to have the calibration sample pass at least twice in the degasser and to analyze the gas composition of the extracted gases of each calibration compound to be analyzed, which requires having available an initial mud sample containing a large amount of calibration compounds, the intention being to evaluate the extraction efficiency thereof. EP-A-2 380 017 performs the calibration using a first group of hydrocarbon calibration compounds and extrapolating the results to a second group of calibration compounds.

The above methods take 3 to 4 hours and require about 20 liters of mud containing a significant amount of hydrocarbons and therefore exiting the well bore after the drilling process. As a result, they can only be performed during a drilling break after a gas peak.

US-A-2014/067307 enables to perform calibration by injecting liquid alkanes into a drilling mud sample in order to prepare a synthetic calibration mud for performing calibration before the beginning of the drilling phase.

All of these methods are time consuming and do not allow performing a calibration each time the properties of the drilling mud coming out of the well change.

The disclosure relates to a method as described above, further comprising:

- performing a second gas extraction in a second extraction device distinct from the first extraction device in order to obtain at least a second extracted gas and a third flow of drilling mud, and measuring at least a second parameter representative of a composition of the second extracted gas and

- using at least the second parameter in order to calculate the correction factor.

Such a method enables to perform the determination of correcting coefficient on a continuous basis, or at least during drilling following a change in the drilling mud, without interrupting the content determination.

The method according to the disclosure may comprise one or more of the following features, taken individually or according to any technically possible combination(s):

- the second gas extraction is performed by injecting at least a second part of the first flow of drilling mud in the second gas extraction device;
- the first extraction device comprises a first enclosure having a first inner volume, and the second extraction device comprises a second enclosure having a second inner volume, wherein the first enclosure and the second enclosure are adapted to receive drilling mud, wherein the second inner volume is smaller than the first inner volume;
- the second gas extraction is performed by injecting the second flow of drilling mud in the second gas extraction device, and the correction factor is calculated using at least the first parameter and the second parameter;
- the method further comprising performing at least one additional gas extraction, wherein said additional extraction includes injecting the third flow of drilling mud in an additional extraction device distinct from the first extraction device and from the second extraction device;
- the method comprises:

    - performing a third gas extraction by injecting the third flow of drilling mud in the second gas extraction device in order to obtain at least a third extracted gas, and
    - measuring at least a third parameter representative of a composition of the third extracted gas, wherein the correction factor is calculated using at least the second parameter and the third parameter;

- injecting the third flow of drilling mud in the second extraction device includes using a mud conduit extending between a mud outlet and a mud inlet of the second extraction device;
- the parameter used for calculating the correction factor is representative of a gas fraction of a calibration compound present in the second extracted gas, the calibration compound being distinct from the drilling compound;
- the first extraction device or the second extraction device comprises an enclosure adapted to receive drilling mud, wherein the enclosure defines an orifice, the method further comprising introducing a mixture of liquid alkanes into the enclosure;
- performing the first gas extraction and performing the second gas extraction include using a same motor in order to stir the first flow of drilling mud and to stir the third flow of drilling mud respectively in the first extraction device and in the second extraction device;
- measuring the first parameter and measuring the second parameter include using a same measuring device;
- the content of the drilling compound contained in the drilling mud is calculated using the following equation:

$$x(i) = \frac{Q_g}{Q_m} \cdot \rho(i) \cdot y_1(i),$$

wherein:

    x (i) is the content of the drilling compound contained in the drilling mud,
    $Q_m$, is a flow rate of the first flow of drilling mud,
    $Q_g$ is a flow rate of the first extracted gas,
    p(i) is the correction factor, and
    $y_1(i)$ is the first parameter; and

- calculating the correction factor includes using the following equation, or an equivalent one:

$$\rho(i) = 1 + Q_m \left( \frac{1}{\alpha(i)\,K(i)V_g} + \frac{1}{\alpha(i)V_m} + \frac{1}{K(i)\,Q_g} \right),$$

wherein:

$V_m$ is an average volume of drilling mud in the first extraction device,
$V_g$ is a volume of gas equal to an internal volume of the first extraction device minus $V_m$,
$\alpha(i)$ and $K(i)$ are parameters relative to the drilling compound and are obtained using:

$$K(i) = a \times \exp(b \cdot F(i)) \text{ and } \alpha(i) = c \times \exp(b \cdot F(i)), \text{ wherein:}$$

wherein:

$\underline{a}$, $\underline{b}$, $\underline{c}$ are adjustment variables determined using at least the second parameter ($y_2(i)$), and
$F(i)$ is a thermodynamic factor using physical parameters of said drilling compound and a given temperature.

The disclosure also relates to an analysis assembly for determining the content of at least one drilling compound contained in a drilling mud, comprising:

- a first extraction device for performing a first gas extraction by injecting at least a first part of a first flow of said drilling mud exiting a wellbore, the first flow being injected in the first extraction device of the first extraction device, in order to obtain a first extracted gas and a second flow of drilling mud exiting the first extraction device,
- a first measuring device for obtaining at least a first parameter representative of a gas fraction of said drilling compound in the first extracted gas, and
- a first calculation unit for calculating said content of the drilling compound using at least the first parameter and a correction factor,

wherein the analysis assembly further comprises:

- a second extraction device distinct from the first extraction device and adapted for performing a second gas extraction in order to obtain a second extracted gas and a third flow of drilling mud, and
- a second measuring device for obtaining at least a second parameter representative of a composition of the second extracted gas, and
- a second calculation unit for calculating the correction factor using at least the second parameter.

The assembly according to the disclosure may comprise one or more of the following features, taken individually or according to any technically possible combination(s):

- an inlet of the second extraction device is in fluid communication with an outlet of the first extraction device for injecting the second flow of drilling mud in the second extraction device and performing the second gas extraction from the second flow of drilling mud and wherein the second calculation unit is configured to calculate the correction factor using at least the first parameter and the second parameter; and
- a mud conduit extends between an outlet and an inlet of the second extraction device for injecting the second flow of drilling mud in the second extraction device and performing a third gas extraction from the third flow of drilling mud in order to obtain at least a third extracted gas, wherein the assembly comprises a third measuring device for obtaining at least a third parameter representative of a composition of the third extracted gas, and wherein the second calculation unit is configured to calculate the correction factor using at least the second parameter and third parameter.

The disclosure will be better understood upon reading the description which follows, given only as an example, and made with reference to the appended drawings, wherein:

- Fig. 1 is a schematic vertical sectional view of a drilling installation comprising an analysis assembly according to a first embodiment of the disclosure;
- Fig. 2 is schematic partial view of the analysis assembly shown in Fig. 1;
- Fig. 3 is a graph illustrating gas fractions of different compounds in gases extracted from a drilling mud in the extraction system shown in Fig. 2; and
- Fig. 4 is schematic partial view of an extraction system according to a second embodiment of the disclosure.

In all the following, the terms "upstream" and "downstream" are to be understood relatively to the normal direction of circulation of a fluid in a conduit.

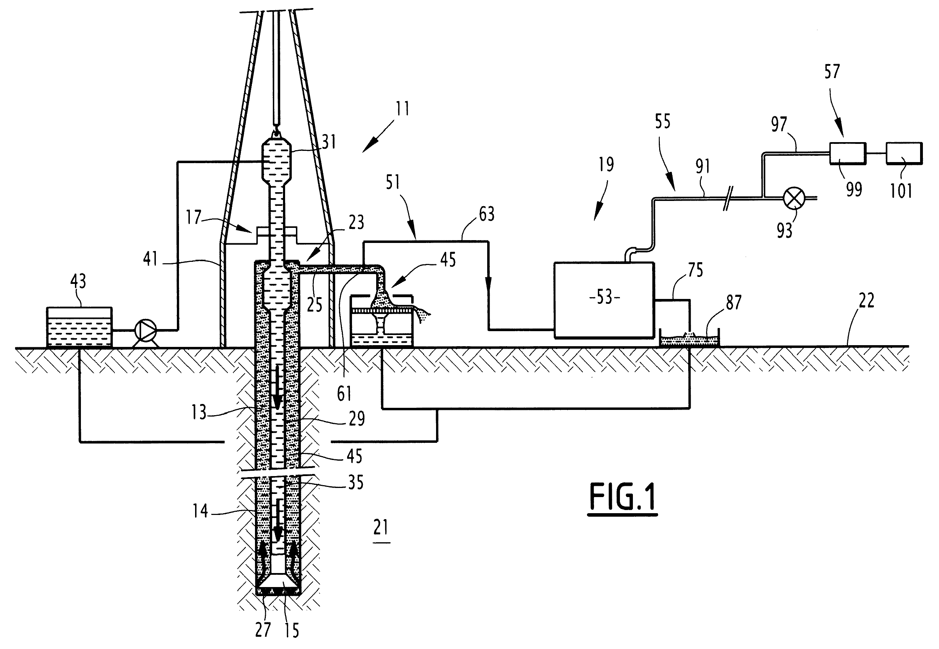

With reference to Fig. 1, a drilling installation 11 is described.

The drilling installation 11 comprises of a well for producing fluid, notably hydrocarbons, such as an oil well.

This installation 11 comprises a drilling conduit 13 positioned in a cavity 14 pierced by a rotary drilling tool 15 in a subsoil 21, a surface installation 17, and an assembly 19 for analyzing the gases contained in the drilling mud exiting the well.

The drilling conduit 13 includes at the surface 22 a well head 23 provided with a conduit 25 for circulation of the fluid.

The drilling tool 15 comprises, from bottom to top in Fig. 1, a drilling head 27, a drill string 29, and a head 31 for injecting drilling fluid. The drilling tool 15, is driven into rotation by the surface installation 17.

The drilling head 27 is mounted on the lower portion of the drill string 29 and is positioned in the bottom of the cavity 14.

The string 29 comprises a set of hollow drilling tubes. These tubes delimit an inner space 35 which allows the drilling fluid injected through the head 31 from the surface 22 to be brought as far as the drilling head 27.

This drilling fluid, commonly designated with the term of « drilling mud », is essentially liquid.

The surface installation 17 comprises a system 41 for supporting and driving into rotation the drilling tool 15, a system 43 for injecting the drilling fluid and a vibrating sieve 45.

The injection system 43 is hydraulically connected to the injection head 31 for introducing and circulating the drilling fluid in the internal space 35 of the drill string 29.

The drilling fluid is introduced into the inner space 35 of the drill string 29 through the injection system 43. This fluid flows downwards down to the drilling head 27 and passes into the drilling conduit 13 through the drilling head 27. This fluid cools and lubricates a drilling head 33. The fluid collects the solid debris resulting from the drilling and flows upwards through the annular space defined between the drill string 29 and the walls of the drilling conduit 13, and is then discharged through the circulation conduit 25.

The inner space 35 opens out facing the drilling head 27 so that the drilling fluid lubricates the drilling head 33 and flows upwards in the cavity 14 along the conduit 13 up to the well head 23, while discharging the collected solid drilling debris, in the annular space 45 defined between the string 29 and the conduit 13.

The circulation conduit 25 is hydraulically connected to the cavity 14, through the well head 23 in order to collect the drilling fluid from the cavity 14. It is for example formed by an open mud conduit or by a closed tubular conduit.

The vibrating sieve 45 collects the fluid loaded with drilling residues which flow out of the circulation conduit 25, and separates the liquid from the solid drilling residues.

The analysis assembly 19 comprises a device 51 for sampling drilling mud in the conduit 25, an extraction system 53 for extracting gases contained in the drilling mud, a transport device 55 for transporting the extracted gases, a measuring device 57 forming in this embodiment the first and second measuring devices, and a computer 101 comprising a first and a second calculation units.

The sampling device 51 comprises a sampling head 61 immersed in the circulation conduit 25, a sampling conduit 63 connected upstream to the sampling head 61.

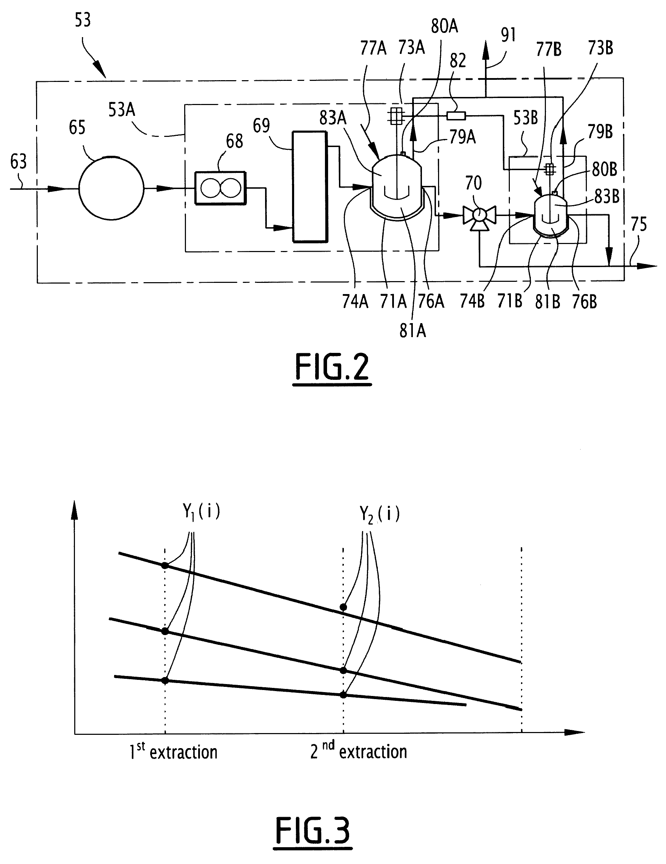

With reference to Fig. 2, the extraction system 53 comprises a pump 65 downstream of the sampling conduit 63, a first extraction device 53A for performing a first gas extraction, a second extraction device 53B for performing a second gas extraction, and a mud discharge conduit 75.

The extraction system 53 also comprises a three-way valve 70, enabling to by-pass the second extraction device 53B, if needed.

The pump 65 is for example a peristaltic pump capable of conveying the drilling mud sampled by the head 61 towards the first extraction device 53 with a determined mud volume flow rate $Q_m$.

The first extraction device 53A comprises a first enclosure 71 A, and a first rotary stirrer 73A mounted in the first enclosure 71A.

The first extraction device 53A may comprise a flow meter 68 and a mud heater 69 hydraulically connected in series between the pump 65 and the first enclosure 71 A.

The first enclosure 71A has a first mud inlet 74A for receiving a first flow of drilling mud from the sampling device 51, and a first mud outlet 76A for delivering a second flow of drilling mud.

The first enclosure 71A also has a first gas inlet 77A for injecting a carrier gas, and a first gas outlet 79A for recovering a first extracted gas.

The first enclosure 71A may define an orifice 80A for introducing a mixture of liquid alkanes into the first enclosure. This orifice is however optional and the method may be performed thanks to drilling mud exiting the wellbore only.

The enclosure 71 has an inner volume for example comprised between 0.04L and 3L. It defines a lower portion 81 containing the drilling fluid stemming from the supply conduit 67 and an upper portion 83 defining a gas head space above the drilling fluid. The volume of mud $V_m$ is kept constant by controlling the flow of drilling fluid $Q_m$ and the volume of the upper portion $V_9$, , corresponding to the volume of the enclosure minus the volume of the mud $V_m$ is therefore kept constant as well. The first mud outlet 76A is connected to the valve 70 and opens out into the lower portion 81 A.

The first gas outlet 79A is connected to the transport device 55.

The first stirrer 73A is immersed into the drilling mud present in the lower portion 81A. It is capable of vigorously stirring the drilling mud in order to extract at least a drilling compound to be analyzed or a calibration compound present in the drilling mud.

In the example shown, the carrier gas is formed by the surrounding air around the installation, at atmospheric pressure. Alternatively, this carrier gas is another gas such as nitrogen or helium.

The second extraction device 53B has a similar structure as the first extraction device 53A and will not be described in details. Similar elements have a similar numeral reference, except that "A" is replaced by "B" in the reference. For example, the second extraction device 53B has a second rotary stirrer 73B. Only the differences will be described in details.

The second extraction device 53B has a first mud inlet 74B connected to the valve 70 for receiving the second flow of drilling mud, and a second mud outlet 76B connected to the discharge conduit 75.

A heater may be disposed upstream from the first and second extraction devices.

The first and second extraction devices 53B may comprise a temperature sensor (not shown) adapted for measuring a temperature inside the extraction devices.

The second enclosure 71 B has a second gas outlet 79B for recovering a second extracted gas.

In a particular embodiment, the second extraction device 53B and the first extraction device 53A are substantially identical and for example have an enclosure geometry which is identical (notably in size or in volume), and an identical stirrer.

The temperature of the drilling mud, the pressure P of the gas head space located above the mud, the drilling mud flow rate $Q_m$ admitted in the enclosures, and the extracted gas flow rate $Q_g$, the volume $V_m$ of drilling mud in the enclosures, the gas volume Vg present in the enclosures, the nature of the stirring as well as the stirring rate, may be substantially identical in the first extraction device 53A and the second extraction device 53B.

The first stirrer 73A and the second stirrer 73B may be driven by a same motor 82.

As a variant (not shown), the analysis assembly 19 has one or several additional extraction device(s) distinct from the first extraction device 53A and from the second extraction device 53B for performing additional gas extractions.

The discharge conduit 75 is connected to a retention tank 87 intended to receive the drilling mud discharged out of the extraction system 53. The discharge conduit 75 is connected to the valve 70, and to the second mud outlet 76B for receiving a third flow of drilling mud exiting the second extraction device 53B.

The transport device 55 comprises a line 91 for transporting the first extracted gas or the second extracted gas towards the measuring device 57, and a suction system 93 for conveying the extracted gases through the transport line 91.

The measuring device 57 comprises a sampling conduit 97 tapped on the transport line 91 upstream from the suction system 93, and an instrumentation 99.

The instrumentation 99 is capable of detecting and quantifying gas fractions in the first extracted gas and in the second extracted gas which are transported through the transport line 91.

This instrumentation 99 for example comprises infrared detection apparatuses for the amount of carbon dioxide, chromatographs with flame ionisation detectors (FID) for detecting hydrocarbons or further with thermal conductivity detectors (TCD) depending on the gases to be analyzed.

It may also comprise a chromatography system coupled with a mass spectrometer, this system being designated by the acronym "GC-MS". It may comprise an isotope analysis apparatus as described in Application EP-A-1 887 343 of the Applicant. The instrumentation may comprise one detection apparatus coupled to several extraction devices or several detection apparatuses, such as each detection apparatus is couples to an extraction device.

Online simultaneous detection and quantification of a plurality of drilling compounds contained in the fluid, without any manual sampling by an operator, is therefore possible within time intervals of less than 1 minute.

The drilling fluid for example is formed by oil-based mud (having oil as a main component) or water-based mud (having water as a main component). In general, drilling mud compounds contain at least hydrocarbons with $C_n$ with n<20. The hydrocarbon compounds that are analyzed are usually up to $C_8$, however higher $C_n$ ones can be analyzed if needed.

A method of determining the content x(i) of several drilling compounds in the drilling fluid according to the disclosure will now be described. An index i is used to identify the drilling compounds. The number of drilling compounds to be analysed is one or several. For example there are five different drilling compounds to be analysed.

The method may use the analysis assembly 19.

The method comprises:

- obtaining a first flow of said drilling mud,

- performing a first gas extraction by injecting at least a first part of the first flow in a first extraction device 53A via a first mud inlet 74A, and extracting gas from the first extraction device in order to obtain a first extracted gas and a second flow of drilling mud exiting the first extraction device, and measuring at least a first parameter $y_1(i)$ representative of a gas fraction of said drilling compound in the first extracted gas,
- using at least the first parameter and a correction factor $p(i)$ in order to calculate said content $x(i)$ of the drilling compound,
- performing a second gas extraction in a second extraction device 53B distinct from the first extraction device in order to obtain at least a second extracted gas and a third flow of drilling mud, and measuring at least a second parameter $y_2(i)$ representative of a composition of the second extracted gas), and
- using at least the second parameter in order to calculate the correction factor $p(i)$.

The second gas extraction is performed by injecting the second flow of drilling mud in the second gas extraction device 53B, and the correction factor $p(i)$ is calculated using at least the first parameter $y_1(i)$ and the second parameter $y_2(i)$.

The first and second parameters $y_1(i)$ and $y_2(i)$ used for calculating the correction factors are relative to at least a same calibration compound. The calibration compound for determining the correcting factor for a predetermined drilling compound may be the predetermined drilling compound, or may be distinct from the predetermined drilling compound. The calibration compound may be another drilling compound for instance or another compound contained in the extracted gas.

When the determination of the correcting factor is performed continuously, the first and second parameters for determining a correcting factor $p(i, T_1)$ and the content $x(i, T_1)$ at a time $T_1$ are the first parameter $y_1(i, T_1)$ at the time $T_1$ and the second parameter $y_2(i, T_2)$ at the time $T_2$, wherein $T_2 - T_1$ corresponds to $\Delta T$ which is the time period that the flow of mud takes for travelling from the first to the second extraction device. The correcting factor may then be determined with increased accuracy. However, as the drilling fluid properties do not vary considerably in a short period of time, such a continuous determination of the correcting factor is not always necessary to obtain an accurate measurement of the content of the drilling compound in the drilling fluid. When the first and second extracted gas are extracted essentially simultaneously, a correcting factor may be calculated accurately.

The first extraction is performed using drilling mud coming directly from the well via the sampling device 51.

The first flow of drilling mud is injected in the first mud outlet 74A. The drilling mud passes through the first enclosure 71A. Thanks to the first stirrer 73A, the compounds are extracted from the drilling mud and are recovered at the first gas outlet 79A in the first extracted gas together with the carrier gas injected in the first gas inlet 77A. The first extraction may be performed when the first extraction device 53A runs in steady state mode, that is to say when the drilling mud within the first enclosure 71A and the first extracted gas have a constant composition versus time.

The first extracted gas is driven to the measuring device 57 by the transport device 55. In this embodiment, it is connected by the transport device to the first and second extraction devices 53A, 53B.

The measuring device 57 constitutes first and second measuring devices as it provides the first parameter $y_1(i)$ and the second parameter $y_2(i)$ for each drilling compound, for example gas fractions. The graph in logarithmic scale represented in Fig. 3 shows the first parameter $y_1(i)$ and the second parameter $y_2(i)$ of three drilling compounds $C_1$ to $C_3$.

If the corrections factors $p(i)$ are known (for example because they were previously determined as will be explained below), the content $x(i)$ of each drilling compound is calculated by the first calculation unit of the computer 101 for example using the following equation:

$$x(i) = \frac{Q_g}{Q_m} \cdot \rho(i) \cdot y_1(i),$$

wherein:

i is the index of the drilling compound,
$Q_m$, is a flow rate of the first flow of drilling mud, and
$Q_g$ is a flow rate of the first extracted gas.

In that case, a second extraction is normally not performed. The valve 70 is switched so that the second flow of drilling mud is directed towards the discharge conduit 75 without going through the second extraction device 53B.

If the corrections factors $p(i)$ are not known, or each time they need to be recalculated, for example due to a change in the drilling mud nature, the second extraction is performed. To that end, the valve 70 is switched so that the second flow of drilling mud, or at least part of it, is injected in the second enclosure 71 B via the second mud inlet 74B.

The second extraction is performed in the same manner as the first extraction and will not be described in details. The second extracted gas is directed into the measuring device 57 in order to obtain the second parameters $y_2(i)$, which are the gas fraction of the compounds in the second extracted gas.

The second parameters $y_2(i)$ are also illustrated in Fig. 3.

As a variant, one or several additional extractions are performed successively on the drilling mud in order to obtain $\underline{n}$ parameters $y_1(i)$, ... $y_n(i)$ representative of gas fractions of each compound, with n being the total number of extraction devices.

When the extraction conditions of the first extraction and the second extraction are similar, the parameters relative to a predetermined drilling compound may be linked by the following equation:

$$y_n(i) = y_1(i) \times \exp\left[-m(i) \times (n-1)\right]$$

The correction factors p(i) for the compound is for example calculated by the second calculation unit of the computer 101 as follows:

$$\rho_i(i) = \frac{1}{y_1(i)} \sum_1^\infty y_n(i) = \frac{1}{1 - \exp(-m(i))} = \frac{1}{1 - \lambda(i)},$$

wherein $\lambda(i) = y_1 (i/ y_2 (i$

In that case, knowing the second parameters $y_2(i)$ and the first parameters $y_1(i)$ for each predetermined compound allows calculating the correction factors p(i) directly.

As a variant, for instance in order to obtain some of the correction factors $\rho(i)$, especially those of heavier compounds such as $C_5$-$C_{10}$ hydrocarbons, another model may be used by the computer 101.

In this variant, at least some of the correction factors p(i) may be calculated using the equation:

$$\rho(i) = 1 + Q_m \left( \frac{1}{\alpha(i)\, K(i) V_g} + \frac{1}{\alpha(i) V_m} + \frac{1}{K(i)\, Q_g} \right) \quad (1),$$

wherein:

$V_m$ is an average volume of drilling mud in the first enclosure 71A,
$V_g$ is a volume of gas equal to an internal volume of the first enclosure 71A minus $V_m$,
$\alpha(i)$ and $K(i)$ are parameters relative to each compound.

The parameters $\alpha(i)$ and $K(i)$ are obtained for example using:

$$K(i) = a \times \exp(b \cdot F(i)) \text{ and } \alpha(i) = c \times \exp(b \cdot F(i)),$$

wherein:

$\underline{a}$, $\underline{b}$, $\underline{c}$ are adjustment variables determined using at least the second parameter, and
$F(i)$ are thermodynamic factors of the compounds respectively depending on physical parameters of the compounds and on the temperature of the drilling mud in the extraction enclosure

Each thermodynamic factor $F(i)$ may be obtained as proposed by *Hoffman* (Hoffman et al. « Equilibrium Constants for a Gas Condensate System » Trans. AIME (1953) 198,1-10) or in an improved way by Standing (Standing, « A set of Equations for Computing Equilibrium Ratios of a Crude Oil/Natural Gas System at Pressures below 1,000 psia » SPE 7903 1979):

$$F(i) = \frac{\left[\dfrac{1}{\theta_b(i)} - \dfrac{1}{\theta}\right]}{\left[\dfrac{1}{\theta_b(i)} - \dfrac{1}{\theta_c(i)}\right]} \cdot \log\left(\frac{P_c(i)}{P_{atm}}\right)$$

wherein:

$\theta$ is the temperature of the drilling fluid in the extraction enclosure,
$\theta_b(i)$ is the boiling temperature of said compound at atmospheric pressure,
$\theta_c(i)$ and $P_c(i)$ are the critical temperature and critical pressure of said compound, and
$P_{atm}$ is the atmospheric pressure.

The adjustment variables a, b, c are the same for each compound. They may be determined as follows.
The above equation (1) may be rewritten as follows for calibration compounds naturally present or introduced in the drilling mud:

$$\rho(j) = 1 + \frac{Q_m}{V_g} \cdot \frac{1}{a \cdot c \times \exp[2b \cdot F(j)]} + \frac{Q_m}{V_m} \cdot \frac{1}{c \times \exp(b \cdot F(j))} + \frac{Q_m}{Q_g} \cdot \frac{1}{a \times \exp(b \cdot F(j))} \quad (2)$$

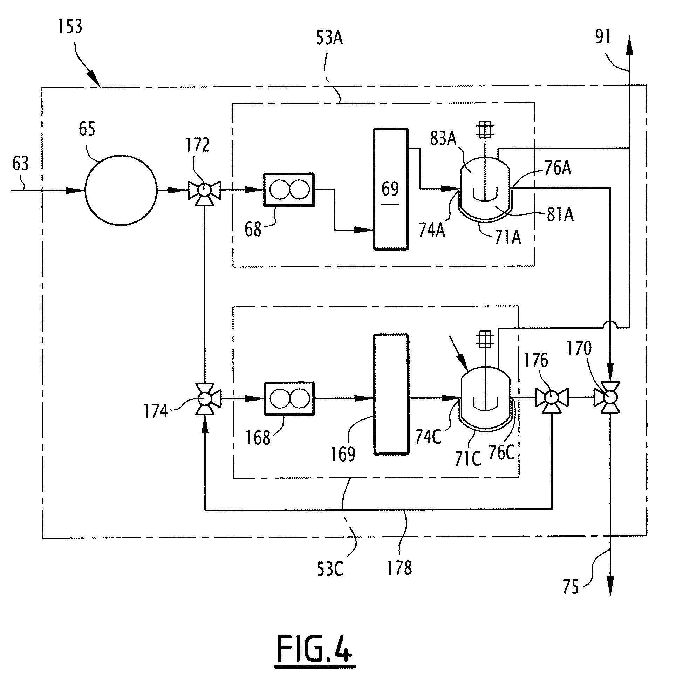

wherein j is an index of a calibration compound.
With at least two calibration compounds and knowing their corrections factors p(j), it is then possible to calculate a, b, c using a least square technique to solve the system.
The parameters $\alpha(i)$ and $K(i)$ are then calculated, and the correction factors p(i) are obtained using the above equation (1).
The calibration compounds may for instance be the $C_1$ to $C_3$ hydrocarbons. The correcting factors for these compounds are obtained using the first and second parameters. Then, a system of three equations (2) are solved thanks to the known values of $\rho(1)$, $\rho(2)$ and $\rho(3)$.
Optionally, liquid alkanes, such as one or several taken from pentane, isopentane, normal-hexane or 2,2-dimethylpentane, are added into the first enclosure 71A in order to include specific calibration compounds in the drilling mud and to get correction factors for drilling compounds in view of the correction factors obtained from the calibration compounds $\rho(j)$.
Thanks to the above features, obtaining the correction factors $\rho(i)$) does not require interrupting the first gas extraction. The first gas extraction is used not only for determining the contents of the drilling compounds in the drilling mud, but also as the first stage of a calibration method having at least two extraction stages, the calibration method being performed on an as needed basis.
With reference to Fig. 4, a second embodiment of the disclosure will be described. In the second embodiment, the extraction system 53 is replaced by an extraction system 153 in the analysis assembly 19 shown in Fig. 1.
The extraction system 153 shown in Fig. 4 is analogous to the extraction system 53 shown in Fig. 2. Similar elements have the same numeral references and will not be described again. Only the differences will be described in details.
The extraction system 153 comprises a second extraction device 53C distinct from the first extraction device 53A and mounted in parallel with the first extraction device 53A, not in series. In the second extraction device 53C, similar elements have similar numeral references as in the first extraction device 53A, except that the letter "A" is replaced by the letter "C".
The second extraction device 53C comprises a second enclosure 71C having a second inner volume. The second inner volume may be smaller than the first inner volume of the first enclosure 71A.
The extraction system 153 comprises a first three-way valve 172 located upstream of the mud inlets 74A, 74C of both extractions devices 53A, 53C, and a second three-way valve 170 located downstream of their mud outlets 76A, 76C. The extraction system 153 also comprises a third and a fourth three-way valves 174, 176, and a mud conduit 178 extending between these three-way valves.
The extraction system 153 runs in a rather similar manner as the extraction system 53.
The first valve 172 allows dividing the first flow of drilling mud into a first part entering the first extraction device 53A, and a second part entering the second extraction device 53C on an as needed basis. Alternatively, the first valve 172 directs the whole first flow of drilling mud exiting the wellbore either to the first extraction device 53A or to the second extraction device 53C.

Like with the extraction system 53, the first extraction is performed in the first extraction device 53A, providing the first extracted gas sent to the measuring device 57, and the second flow of drilling mud. The second flow of drilling mud is then evacuated via the valves 176 and 170 in the discharge conduit 75. The first extraction provides the first parameter $y_1(i)$ for each drilling compound to be measured.

The second extraction provides the second extracted gas sent to the measuring device, and the third flow of drilling mud collected at the mud outlet 76C. The second parameter $y_2(i)$ is obtained for each drilling compound from the measuring device 57.

As a variant, the second parameter $y_2(i)$ is obtained for one or several calibration compounds that are different from the drilling compounds.

The third flow of drilling mud is reinjected in the enclosure 71C via the valve 176, the mud conduit 178 and the valve 174. A third extraction is then performed in the second extraction device 53C in order to obtain a third extracted gas and a fourth flow of drilling mud.

Analysis of the third extracted gas by the measuring device 57 provides a third parameter $y_3(i)$ for each drilling compound, or for the calibration compound(s). The fourth flow of drilling mud is evacuated from the second extraction device 53C via the valves 176,170.

It will be noted that in this embodiment the measuring device 57 constitutes the first measuring device for determining the first parameter $y_1(i)$, the second measuring device for determining the second parameter $y_2(i)$ and the third measuring device for determining the third parameter $y_3(i)$. However, the first, second and third measuring devices may be formed by different sensors or devices.

In particular embodiments, the fourth flow of drilling mud is not evacuated, but reinjected again in the second extraction device 53C or in another extraction device (not represented) for a fourth extraction. More extractions may be performed in some embodiments.

Using at least the second parameter $y_2(i)$ and the third parameter $y_3(i)$, the corrections factor p(i) is calculated for each drilling compound or calibration compound. In the latter case, the correction factor $\rho(i)$ for each of the drilling compounds is derived from the correction factors of the calibration compounds, as described in connection with the first embodiment.

Based on the correction factor $\rho(i)$ and the first parameter $y_1(i)$, the content x(i) of each drilling compound is calculated.

The extraction system 153 brings the same advantages as the extraction device 53. It also enables to make the gas extractions used to determine the correction factor $\rho(i)$ separate from the first extraction.

**Claims**

1.  Method of determining the content (x(i)) of at least one drilling compound contained in a drilling mud, the method comprising:

    - obtaining a first flow of said drilling mud exiting a wellbore,
    - performing a first gas extraction by injecting at least a first part of the first flow in a first extraction device (53A) and extracting gas from the first extraction device (53A) in order to obtain a first extracted gas and a second flow of drilling mud exiting the first extraction device (53A), and measuring at least a first parameter ($y_1(i)$) representative of a gas fraction of said drilling compound in the first extracted gas, and
    - using at least the first parameter ($y_1(i)$) and a correction factor ($\rho(i)$) in order to calculate said content (x(i)) of the drilling compound,

    wherein the method further comprises:

    - performing a second gas extraction in a second extraction device (53B; 53C) distinct from the first extraction device (53A) in order to obtain at least a second extracted gas and a third flow of drilling mud, and measuring at least a second parameter ($y_2(i)$) representative of a composition of the second extracted gas, and
    - using at least the second parameter ($y_2(i)$) in order to calculate the correction factor ($\rho(i)$).

2.  Method according to claim 1, wherein the second gas extraction is performed by injecting at least a second part of the first flow of drilling mud in the second gas extraction device (53C).

3.  Method according to claim 2, wherein the first extraction device (53A) comprises a first enclosure (71A) having a first inner volume, and the second extraction (53C) device comprises a second enclosure (71 C) having a second inner volume, wherein the first enclosure (71A) and the second enclosure (71 C) are adapted to receive drilling mud, wherein the second inner volume is smaller than the first inner volume.

4. Method according to claim 1, wherein the second gas extraction is performed by injecting the second flow of drilling mud in the second gas extraction device (53B), and the correction factor ($\rho(i)$) is calculated using at least the first parameter ($y_1(i)$) and the second parameter ($y_2(i)$).

5. Method according to claim 4, further comprising performing at least one additional gas extraction, wherein said additional extraction includes injecting the third flow of drilling mud in an additional extraction device distinct from the first extraction device (53A) and from the second extraction device (53B).

6. Method according to any of claims 1 to 5, wherein the method comprises:

    - performing a third gas extraction by injecting the third flow of drilling mud in the second gas extraction device (53B) in order to obtain at least a third extracted gas, and
    - measuring at least a third parameter ($y_3(i)$) representative of a composition of the third extracted gas, wherein the correction factor ($\rho(i)$) is calculated using at least the second parameter ($y_2(i)$) and the third parameter($y_3(i)$).

7. Method according to claim 6, wherein injecting the third flow of drilling mud in the second extraction device (53B) includes using a mud conduit (178) extending between a mud outlet (76C) and a mud inlet (74A) of the second extraction device (53C).

8. Method according to any of claims 1 to 7, wherein the parameter ($y_2(i)$ used for calculating the correction factor ($\rho(i)$) is representative of a gas fraction of a calibration compound present in the second extracted gas, the calibration compound being distinct from the drilling compound.

9. Method according to any of claims 1 to 8, wherein the first extraction device (53A) or the second extraction device (53B) comprises an enclosure (71 A, 71 B) adapted to receive drilling mud, wherein the enclosure (71A, 71 B) defines an orifice (80A, 80B), the method further comprising introducing a mixture of liquid alkanes into the enclosure (71A, 71 B).

10. Method according to any of claims 1 to 9, wherein performing the first gas extraction and performing the second gas extraction include using a same motor (82) in order to stir the first flow of drilling mud and to stir the third flow of drilling mud respectively in the first extraction device (53A) and in the second extraction device( (53B).

11. Method according to any of claims 1 to 10, wherein measuring the first parameter ($y_1(i)$) and measuring the second parameter ($y_2(i)$) include using a same measuring device (57).

12. Method according to any of claims 1 to 11, wherein the content ($x(i)$) of the drilling compound contained in the drilling mud is calculated using the following equation:

$$x(i) = \frac{Q_g}{Q_m} \cdot \rho(i) \cdot y_1(i),$$

wherein:

    $x(i)$ is the content of the drilling compound contained in the drilling mud, $Q_m$, is a flow rate of the first flow of drilling mud,
    $Q_g$ is a flow rate of the first extracted gas,
    $\rho(i)$ is the correction factor, and
    $y_1(i)$ is the first parameter.

13. Method according to claim 12, wherein calculating the correction factor ($\rho(i)$) includes using the following equation, or an equivalent one:

$$\rho(i) = 1 + Q_m \left( \frac{1}{\alpha(i)\, K(i)\, V_g} + \frac{1}{\alpha(i)\, V_m} + \frac{1}{K(i)\, Q_g} \right),$$

wherein:

$V_m$ is an average volume of drilling mud in the first extraction device (53A),
$V_g$ is a volume of gas equal to an internal volume of the first extraction device (53A) minus $V_m$,
$\alpha(i)$ and $K(i)$ are parameters relative to the drilling compound and are obtained using:

$$K(i) = a \times \exp(b \cdot F(i)) \ \text{ and } \ \alpha(i) = c \times \exp(b \cdot F(i)),$$

wherein:

$\underline{a}$, $\underline{b}$, $\underline{c}$ are adjustment variables determined using at least the second parameter ($y_2(i)$), and
$F(i)$ is a thermodynamic factor using physical parameters of said drilling compound and a given temperature.

14. Analysis assembly (19) for determining the content ($x_0(i)$) of at least one drilling compound contained in a drilling mud, comprising:

- a first extraction device (53A) for performing a first gas extraction by injecting at least a first part of a first flow of said drilling mud exiting a wellbore, the first flow being injected in the first extraction device (53A) of the first extraction device (53A), in order to obtain a first extracted gas and a second flow of drilling mud exiting the first extraction device (53A),
- a first measuring device (57) for obtaining at least a first parameter ($y_1(i)$) representative of a gas fraction of said drilling compound in the first extracted gas, and
- a first calculation unit (101) for calculating said content ($x_0(i)$) of the drilling compound using at least the first parameter ($y_1(i)$) and a correction factor ($\rho(i)$),

wherein the analysis assembly (19) further comprises:

- a second extraction device (53B; 53C) distinct from the first extraction device (53A) and adapted for performing a second gas extraction in order to obtain a second extracted gas and a third flow of drilling mud, and
- a second measuring device (57) for obtaining at least a second parameter ($y_2(i)$) representative of a composition of the second extracted gas, and
- a second calculation unit (101) for calculating the correction factor ($\rho(i)$) using at least the second parameter ($y_2(i)$).

15. The analysis assembly according to claim 14, wherein an inlet (74B) of the second extraction device (53B) is in fluid communication with an outlet (76A) of the first extraction device (53A) for injecting the second flow of drilling mud in the second extraction device (53B) and performing the second gas extraction from the second flow of drilling mud and wherein the second calculation unit is configured to calculate the correction factor ($\rho(i)$) using at least the first parameter ($y_1(i)$) and the second parameter ($y_2(i)$).

16. The analysis assembly according to claim 14, wherein a mud conduit (178) extends between an outlet (76C) and an inlet (74C) of the second extraction device (53C) for injecting the second flow of drilling mud in the second extraction device (53C) and performing a third gas extraction from the third flow of drilling mud in order to obtain at least a third extracted gas, wherein the assembly comprises a third measuring device for obtaining at least a third parameter ($y_3(i)$) representative of a composition of the third extracted gas, and wherein the second calculation unit is configured to calculate the correction factor ($\rho(i)$) using at least the second parameter ($y_2(i)$) and third parameter ($y_3(i)$).

**FIG.1**

## FIG.2

$Y_1(i)$    $Y_2(i)$

1$^{st}$ extraction    2$^{nd}$ extraction

## FIG.3

FIG.4

Europäisches
Patentamt

European
Patent Office

Office européen
des brevets

# EUROPEAN SEARCH REPORT

Application Number

EP 15 29 0314

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 703 597 A1 (GEOSERVICES EQUIPEMENTS [FR]) 5 March 2014 (2014-03-05) * paragraphs [0013], [0014], [0019], [0024] * * paragraph [0045] - paragraph [0086]; figures 1, 2 * * paragraph [0115] - paragraph [0162]; claims 1, 8; figures 3-6 * | 1-3, 6-14,16 | INV. G01N33/28 |
| X,D | US 2006/224333 A1 (FRECHIN NICOLAS [FR] ET AL) 5 October 2006 (2006-10-05) * paragraph [0012] - paragraph [0026] * * paragraph [0045] - paragraph [0112]; figures 1-6 * | 1,2,6,7, 10,11,14 | |
| A | US 2011/303463 A1 (LESSI JACQUES [FR]) 15 December 2011 (2011-12-15) * the whole document * | 1-16 | |

TECHNICAL FIELDS
SEARCHED (IPC)

G01N

The present search report has been drawn up for all claims

| Place of search | Date of completion of the search | Examiner |
|---|---|---|
| The Hague | 22 March 2016 | Pagels, Marcel |

## ANNEX TO THE EUROPEAN SEARCH REPORT
## ON EUROPEAN PATENT APPLICATION NO.

EP 15 29 0314

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2016

| Patent document cited in search report | | | Publication date | Patent family member(s) | | Publication date |
|---|---|---|---|---|---|---|
| EP 2703597 | A1 | | 05-03-2014 | EP 2703597 A1<br>US 2014067307 A1<br>WO 2014033265 A1 | | 05-03-2014<br>06-03-2014<br>06-03-2014 |
| US 2006224333 | A1 | | 05-10-2006 | AT 491154 T<br>CA 2541623 A1<br>EP 1710575 A2<br>FR 2883916 A1<br>NO 327508 B1<br>US 2006224333 A1 | | 15-12-2010<br>04-10-2006<br>11-10-2006<br>06-10-2006<br>27-07-2009<br>05-10-2006 |
| US 2011303463 | A1 | | 15-12-2011 | AU 2010205535 A1<br>CA 2749387 A1<br>EP 2380017 A2<br>FR 2941261 A1<br>US 2011303463 A1<br>WO 2010081981 A2 | | 08-09-2011<br>22-07-2010<br>26-10-2011<br>23-07-2010<br>15-12-2011<br>22-07-2010 |

For more details about this annex : see Official Journal of the European Patent Office, No. 12/82

**EP 3 182 119 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2799790
- EP 1710575 A
- EP 2380017 A
- US 2014067307 A
- EP 1887343 A

### Non-patent literature cited in the description

- **HOFFMAN et al.** Equilibrium Constants for a Gas Condensate System. *Trans. AIME,* 1953, vol. 198, 1-10
- **STANDING.** A set of Equations for Computing Equilibrium Ratios of a Crude Oil/Natural Gas System at Pressures below 1,000 psia. *SPE,* 1979, 7903